# FASCICULE DE BREVET EUROPEEN

(11) **EP 3 061 431 B1**
(45) Date de publication et mention de la délivrance du brevet: **25.10.2017**
(21) Numéro de dépôt: 16000288.7
(22) Date de dépôt: 05.02.2016
(51) Int. Cl.: A61F 9/02

(54) **LUNETTES DE SPORT**
SPORTBRILLEN
SPORTS GOGGLES

(30) Priorité: 27.02.2015 FR 1500379
(43) Date de publication de la demande: 31.08.2016
(73) Titulaire: Salomon S.A.S., 74370 Metz-Tessy (FR)
(72) Inventeur: Favre-Felix, Hervé, 74370 Villaz (FR)

(56) Documents cités:
- EP-A1- 0 167 422
- EP-A1- 2 380 538
- WO-A1-99/11207
- WO-A2-2004/031839
- DE-C- 858 289
- US-A- 1 677 747
- US-A- 1 942 394

## Description

L'invention concerne les masques de protection oculaire, en particulier les masques de protection pour la pratique de sports en plein air, tels que le ski ou surf alpin, le vélo tout terrain ou le motocross.

Des masques de protection sont habituellement utilisés durant la pratique de certains sports de plein air pour protéger les yeux de l'utilisateur du vent, de l'eau, de la boue ou du rayonnement solaire.

Un tel masque comprend généralement un châssis supportant un écran de protection transparent et une sangle fixée de part et d'autre du châssis. La sangle permet de maintenir le masque sur le visage en entourant partiellement le crâne de l'utilisateur. Des éléments de confort sont habituellement ajoutés au châssis pour améliorer le confort du port du masque. Ces éléments de confort sont placés à l'interface entre le châssis et le visage. Ils forment souvent une bande de mousse entourant les yeux en prenant appui sur une partie du front, les tempes, les pommettes et une partie du nez de l'utilisateur.

Généralement, le châssis comprend une paroi arrière continue entourant les deux yeux. Cette paroi arrière comprend une face postérieure continue faisant face au visage. Une mousse est habituellement fixée sur cette face postérieure. Cette mousse constitue l'élément de confort décrit précédemment. D'autre part, le châssis comprend avantageusement des moyens élastiques reliant la paroi arrière avec une paroi avant supportant l'écran. Ces moyens élastiques permettent une déformation de la paroi arrière en direction de l'écran. La paroi arrière peut alors mieux s'adapter à la morphologie du visage ce qui améliore également le confort de portage.

Le document EP 2 380 538 propose une telle construction en décrivant un masque comprenant une paroi arrière continue reliée à une paroi avant par des éléments élastiques.

Les constructions de masque de l'art antérieur présentent des moyens d'adaptation morphologique permettant une mobilité de la paroi arrière continue d'un châssis. Or, cette paroi arrière peut présenter une certaine rigidité qui peut pénaliser la conformation du châssis au relief du visage. D'autre part, cette construction propose une répartition d'appui sur le visage sensiblement uniforme sur tout le pourtour des yeux du fait de la continuité de la paroi arrière. La pression de contact exercée par la paroi arrière sur le visage présente une continuité ne permettant pas de grande variation de pression dans des zones d'appui proches. On ne peut donc pas obtenir une pression complètement adaptée en fonction de zones d'appui visage. L'adaptation morphologique est alors limitée ce qui réduit la polyvalence d'utilisation du masque en fonction de la nature du visage de l'utilisateur. De plus, l'unicité de la face postérieure induit des frottements de contact pouvant être inconfortables dès lors que l'utilisateur bouge une partie de son visage, par exemple, un froncement de front ou du nez, sans bouger une autre partie du visage, par exemple, les pommettes ou les tempes.

Le but de l'invention est de proposer un masque amélioré permettant un meilleur confort de portage.

Un but est notamment de mieux s'adapter aux différentes morphologies des individus.

Un autre but est d'avoir une répartition différentielle de la pression de maintien du masque sur le visage sur des zones définies.

Un autre but est de diminuer la pression exercée sur des zones déterminées.

L'invention propose un masque de protection oculaire pour la pratique de sport en plein air comprenant :
- un écran,
- un châssis comprenant une partie antérieure reliée à l'écran et une partie postérieure.

Le masque est caractérisé par le fait que la partie postérieure du châssis comprend au moins deux surfaces postérieures distinctes, chaque surface postérieure formant la face arrière d'une paroi postérieure sensiblement verticale, les parois postérieures étant espacées entre elles par un intervalle s'étendant, en direction de la partie antérieure du châssis, d'une profondeur au moins égale à l'épaisseur des parois postérieures délimitant l'intervalle.

Grâce à cette construction, au moins deux parties distinctes de la partie postérieure peuvent se déformer ou se déplacer indépendamment l'une de l'autre. Cette différenciation de mobilité permet une meilleure adaptation morphologique. La souplesse du châssis liée à cette conception permet de réduire la pression de contact dans des zones localisée.

Selon des aspects avantageux mais non obligatoires de l'invention, un tel masque peut incorporer une ou plusieurs des caractéristiques suivantes, prises dans toute combinaison techniquement admissible :
- La partie postérieure supporte au moins un élément de confort.
- Chaque surface postérieure supporte un élément de confort distinct.
- Un même élément de confort recouvre plusieurs surfaces postérieures.
- Une surface postérieure forme uniquement une partie postérieure supérieure du châssis dont la largeur est au moins égale à la moitié de la largeur du châssis, ladite surface postérieure couvrant uniquement une partie du front.
- La partie postérieure comprend au moins trois surfaces postérieures distinctes agencées de la manière suivante : une surface supérieure couvrant une partie du front, une surface latérale gauche et une surface latérale droite couvrant une partie des pommettes.
- Le châssis est unitaire et composé d'un matériau dont la dureté est supérieure à 70 Shore A.
- Le châssis est réalisé en PolyUréthane.
- Au moins un intervalle séparant deux parois postérieures s'étend d'une profondeur au moins supérieure au quart de l'épaisseur du châssis, au niveau de cet intervalle.
- La partie antérieure et la partie postérieure du châssis sont reliées par des éléments de liaison dimensionnés de sorte à permettre le déplacement ou la déformation d'au moins une surface postérieure indépendamment des autres surfaces postérieures.
- Les moyens de liaison sont dimensionnés de sorte que l'amplitude du déplacement ou de la déformation d'au moins une surface postérieure est différente de celle d'une autre surface postérieure.
- Les éléments de liaison se présentent sous la forme d'un ensemble de poutres, les poutres étant inclinées par rapport à une direction linéaire normale à une surface postérieure et/ou formant une courbe ou un arc de cercle.
- Les éléments de liaison sont agencés de sorte à permettre un pivotement de la paroi postérieure sensiblement autour de son axe longitudinal.
- Chaque surface postérieure est allongée et sa longueur est au moins quatre fois supérieure à sa hauteur.
- L'épaisseur de chaque paroi postérieure est au moins quatre fois inférieure à la hauteur de cette paroi postérieure.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :
- la figure 1 est une vue en perspective avant du masque selon un premier mode de réalisation de l'invention ;
- la figure 2 est une vue en perspective arrière du masque sans sangle muni de plusieurs éléments de confort ;
- la figure 3 est une vue en perspective arrière du masque sans sangle muni d'un seul élément de confort ;

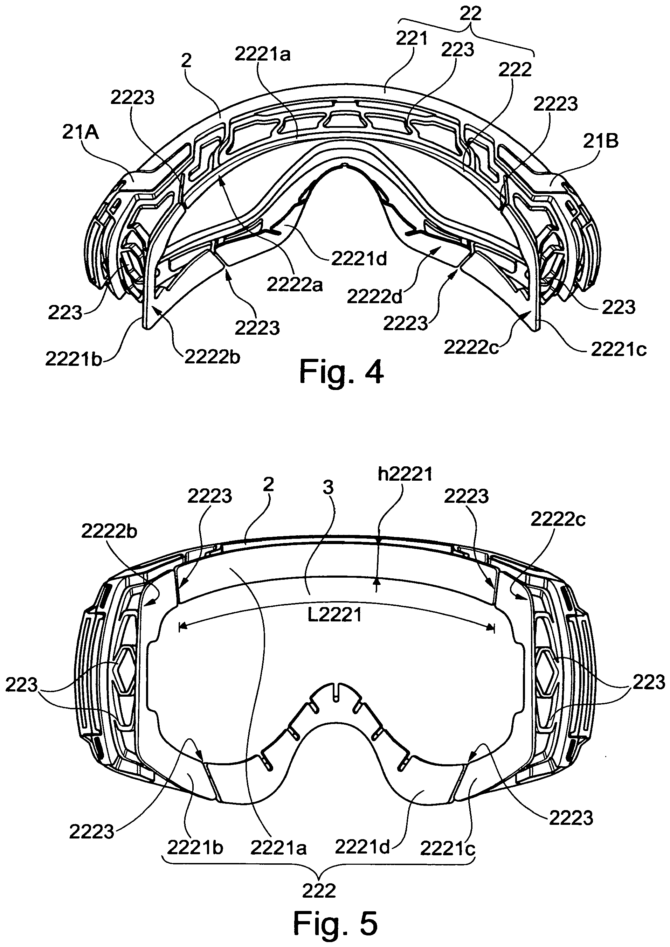

- la figure 4 est une vue en perspective arrière du masque sans sangle et sans élément de confort ;
- la figure 5 est une vue arrière du masque sans sangle et sans élément de confort ;

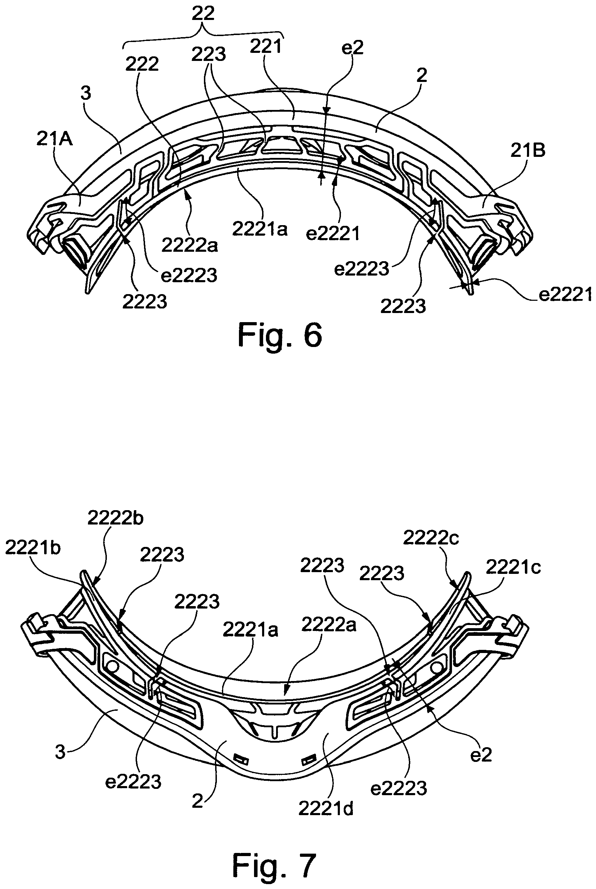

- la figure 6 est une vue de dessus du masque sans sangle et sans élément de confort ;
- la figure 7 est une vue de dessous du masque sans sangle et sans élément de confort.

Le masque 1 comprend un châssis 2 supportant un écran de protection oculaire 3. Une sangle 4 est fixée aux extrémités latérales du châssis 2. La sangle 4 et le châssis 2 forment une boucle destinée à entourer le crâne de l'utilisateur pour assurer le maintien en place du masque.

Dans la suite de la description, il sera fait usage de termes tels que « horizontal », « vertical », « supérieur », « inférieur », « haut », « bas », « avant », « arrière », « devant », « derrière », « antérieur », « postérieur ». Ces termes doivent être interprétés, de façon relative, en relation avec la position normale que le masque occupe sur le visage de l'utilisateur quand il se tient debout, tête droite. Par « arrière » ou « postérieure », on désigne les parties orientées côté tête / yeux de l'utilisateur.

Dans cet exemple, le châssis 2 comprend un corps 22 et deux attaches latérales 21A, 21B. Chaque attache latérale est fixée sur un bord latéral du corps, de part et d'autre de l'écran 3. Chaque extrémité de la sangle 4 est solidaire d'une attache latérale.

Le corps 22 comprend une partie antérieure 221 et une partie postérieure 222. Ces deux parties sont reliées entre elles par des éléments de liaison 223.

La partie antérieure 221 supporte l'écran 3 et les deux attaches latérales 21A, 21B. L'écran peut être assemblé de manière amovible ou non à la partie antérieure. Dans une variante, le châssis 2 comprend un cadre rigide supportant l'écran. Dans ce cas, la partie antérieure du châssis est alors fixée sur ce cadre rigide. Les attaches latérales peuvent être une partie du cadre rigide ou, alternativement, une pièce rapportée au cadre rigide ou au châssis.

La partie postérieure 222 est agencée à l'arrière du châssis. Elle constitue la partie du châssis la plus proche du visage de l'utilisateur lorsque le masque est porté. Selon l'invention, la partie postérieure comprend au moins deux parois postérieures 2221a, 2221b, 2221c distinctes. Dans cet exemple, la partie postérieure est composée de quatre parois postérieures distinctes. Une première paroi postérieure supérieure 2221a recouvre une partie du front. Une deuxième paroi postérieure latérale gauche 2221b et une troisième paroi postérieure latérale droite 2221c recouvrent respectivement une partie des pommettes gauche et droite de l'utilisateur. Une quatrième paroi postérieure inférieure 2221d recouvre le dessus du nez. Chaque paroi postérieure comprend une surface postérieure 2222a, 2222b, 2222c, 2222d formant la face arrière de la paroi postérieure associée.

Chaque paroi postérieure ou la plupart des parois postérieures est sensiblement verticale lorsque le masque est porté, tête droite. Une paroi postérieure est « sensiblement verticale » lorsque sa surface postérieure, non sollicitée, est agencée de sorte que la direction normale à chaque point de la surface postérieure est orientée par rapport à un plan horizontal d'un angle compris entre 0° et 30°.

Localement, une paroi postérieure peut ne pas être « verticale » pour mieux s'adapter à la morphologie de l'utilisateur. Par exemple, ce peut être le cas pour épouser la forme du nez. Dans notre mode de réalisation, la quatrième paroi postérieure inférieure 2221d, couvrant le dessus du nez, peut comprendre des zones de la paroi postérieure plus inclinées.

Avantageusement, chaque surface postérieure n'est pas plane mais courbe gauche afin de mieux se conformer à la morphologie du visage. Par exemple, la surface postérieure supérieure 2221a forme un cylindre autour d'un axe sensiblement vertical pour suivre la courbure du front, dans un plan horizontal. En variante, elle peut être légèrement bombée.

Chaque surface postérieure forme une surface d'appui du châssis en direction du visage. Dans cet exemple, chaque surface postérieure est allongée et sa longueur L2221 est au moins quatre fois supérieure à sa hauteur h2221. Cela permet un appui stable et une bonne répartition de la pression sur une zone délimitée. La surface postérieure peut être rectangulaire, comme l'est la surface postérieure de la première paroi postérieure supérieure 2221a. La surface postérieure peut aussi ne pas être rectiligne et suivre une courbe, formant une parenthèse, comme le sont les surfaces postérieures des deuxième paroi postérieure latérale gauche 2221b et troisième paroi postérieure latérale droite 2221c, ou formant un « V » pour la quatrième paroi postérieure inférieure 2221d.

Chaque paroi postérieure 2221a, 2221b, 2221c forme une bande allongée de faible épaisseur e2221. Dans cet exemple, l'épaisseur de chaque paroi postérieure est au moins quatre fois inférieure à la hauteur de cette paroi postérieure. Selon un mode de réalisation, l'épaisseur e2221 est sensiblement constante. Alternativement, l'épaisseur est variable.

Les parois postérieures 2221a, 2221b, 2221c, 2221d sont agencées de sorte que les surfaces postérieures associées 2222a, 2222b, 2222c, 2222d forment une boucle discontinue entourant les deux yeux en recouvrant une partie du front, une partie des tempes et des pommettes et le dessus du nez. L'ouverture délimitée par cette boucle discontinue est suffisamment grande pour ne pas pénaliser le champ de vision.

Chaque surface postérieure est destinée à venir en appui sur le visage, soit directement, soit par l'intermédiaire d'un élément de confort 5. Pour améliorer le confort de portage, il est préférable d'ajouter un élément de confort. Dans ce cas, l'élément de confort est supporté par les surfaces postérieures. Il s'agit généralement d'une mousse collée sur les surfaces postérieures. La mousse peut être en PolyUréthane (PU), en PolyEthylène (PE), en Ethylène-Acétate de Vinyle (EVA). Alternativement, l'élément de confort peut être un tissu, une poche d'air ou un autre élément adapté. L'accroche de l'élément de confort peut être réalisé autrement que par de la colle, par exemple par soudure ou bi-injection. L'élément de confort peut être aussi amovible. Dans ce cas, l'accroche de l'élément de confort peut être réalisée par des crochets, des clips ou un autre moyen.

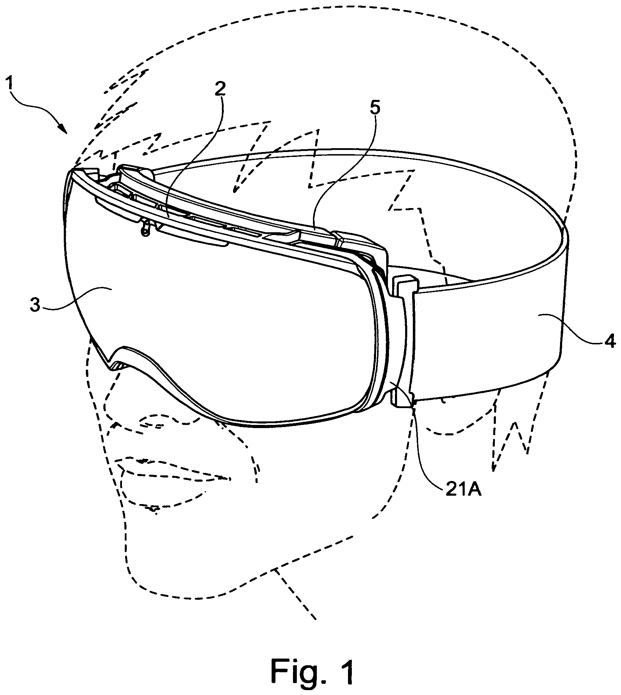

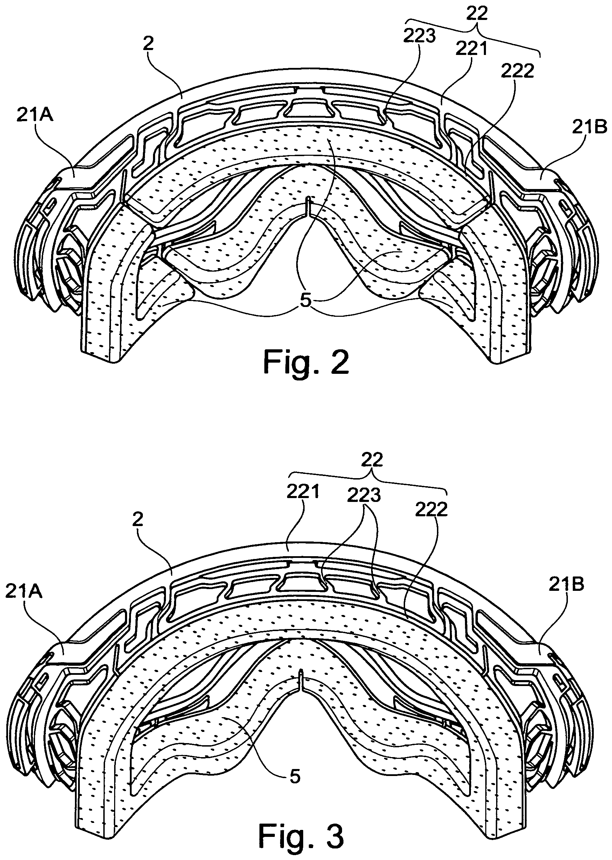

L'élément de confort peut comprendre plusieurs pièces distinctes, chaque pièce distincte étant associée à une seule surface postérieure, comme représenté aux figures 1 et 2. Une pièce distincte peut être une mousse comme on l'a vu précédemment. Dans ce cas, deux mousses voisines sont séparées par un intervalle correspondant sensiblement à l'intervalle séparant les surfaces postérieures supportant les mousses voisines. Cette construction améliore l'adaptation morphologique du masque car chaque surface postérieure équipée d'une pièce distincte de l'élément de confort est mobile indépendamment des autres surfaces postérieures. Le déplacement ou la déformation d'une surface postérieure n'entraîne pas de mouvement des surfaces postérieures voisines.

Alternativement, comme représenté à la figure 3, un même élément de confort recouvre plusieurs surfaces postérieures. Dans cet exemple, le même élément de confort constitue une pièce unitaire recouvrant toutes les surfaces postérieures. Cette construction présente l'avantage d'être plus simple à fabriquer. On a besoin d'un seul élément de confort et celui-ci est plus facile à assembler sur le châssis. Par ailleurs, cela permet une bonne tenue de la partie arrière du châssis quand le masque n'est pas porté et une meilleure isolation du volume entre l'écran et le visage.

L'invention porte sur le fait que les surfaces postérieures, en interface avec le visage ou un élément de confort, forment un pourtour discontinue. Pour cela, les parois postérieures 2221a, 2221b, 2221c supportant les surfaces postérieures 2222a, 2222b, 2222c sont espacées entre elles par un intervalle 2223 s'étendant, en direction de la partie antérieure 221 du châssis, d'une profondeur e2223 au moins égale, et avantageusement supérieure, à l'épaisseur e2221 de la paroi postérieure 2221a, 2221b, 2221c. Autrement dit, deux parois postérieures voisines sont séparées, l'une de l'autre, par un évidement dont la profondeur e2223 est au moins égale à l'épaisseur maximale e2221 des parois postérieures. L'intervalle 2223 entre deux parois postérieures s'étend sur toute la hauteur h2221 des parois postérieures. Cette séparation entre les deux parois postérieures voisines permet un débattement différent d'une paroi postérieure par rapport à l'autre ce qui améliore l'adaptation morphologique.

Pour améliorer le degré de liberté de mouvement d'une paroi postérieure par rapport à une paroi postérieure voisine, il suffit d'augmenter la profondeur e2223 de l'intervalle 2223. En conséquence, avec une profondeur e2223 de l'intervalle 2223 au moins supérieure au quart de l'épaisseur e2 du châssis, au niveau de cet intervalle, les surfaces postérieures ont davantage de mobilité entre elles, ce qui améliore le confort de portage, quelle que soit la forme du visage de l'utilisateur. L'intervalle ne s'étend pas nécessairement de manière rectiligne.

La mobilité des parois postérieures 2221a, 2221b, 2221c, 2221d dépend également de leur liaison avec la partie antérieure 221 du châssis 2. En conséquence, les éléments de liaison 223 contribuent fortement au degré de liberté des parois postérieures 2221a, 2221b, 2221c, 2221d formant la partie postérieure 222 du châssis. Ainsi, le dimensionnement de ces éléments de liaison permet d'avoir des parois postérieures mobiles ou, à l'inverse, un peu plus rigides, en fonction des zones d'appuis visage concernées.

Dans notre exemple, les éléments de liaison 223 se présentent sous la forme d'un ensemble de poutres reliant la partie antérieure à la partie postérieure. Les poutres sont orientées de sorte à faciliter le déplacement des parois postérieures en direction de la partie antérieure. Pour cela, les poutres ne relient pas directement la partie antérieure à la partie postérieure selon une direction linéaire normale aux surfaces postérieures correspondantes mais les poutres sont inclinées par rapport à ces directions normales. Ainsi, lorsqu'une pression est exercée sur une paroi postérieure, du fait de cet agencement des poutres, cette pression génère un effort latéral sur les poutres qui peuvent alors facilement fléchir. Les poutres sont suffisamment espacées pour créer des ouvertures. Celles-ci facilitent la déformation des poutres. Une poutre déformée ne vient pas en contact avec une autre poutre. De plus, ces ouvertures permettent d'aérer le volume intérieur du masque, entre l'écran et le visage.

Selon un mode de réalisation, les poutres forment une courbe ou un arc de cercle, comme c'est le cas pour les parois postérieures latérales 2221b, 2221c. Cette orientation facilite également la déformation des poutres permettant le déplacement des parois postérieures latérales en direction de l'écran.

Le dimensionnement, l'orientation, l'agencement et le nombre de poutres formant les éléments de liaison caractérisent ainsi la mobilité de la paroi postérieure associée. En conséquence, chaque paroi postérieure peut avoir une mobilité différente d'une paroi postérieure voisine. Cette variation de mobilité est également due à l'intervalle séparant deux parois postérieures voisines. Les éléments de liaison peuvent être dimensionnés de sorte à permettre le déplacement ou la déformation d'au moins une surface postérieure indépendamment des autres surfaces postérieures. De plus, les moyens de liaison peuvent être dimensionnés de sorte que l'amplitude du déplacement ou de la déformation d'au moins une surface postérieure est différente de celle d'une autre surface postérieure. Cela permet d'avoir des pressions de contact différentes dans les différentes zones d'appui visage. La variation d'amplitude améliore également l'adaptation morphologique du châssis.

Les éléments de liaisons peuvent également être agencés de sorte à permettre un pivotement de la paroi postérieure sensiblement autour de son axe longitudinal. Par exemple, pour la première paroi postérieure supérieure 2221a, l'ensemble des poutres formant les éléments de liaison sont sensiblement alignés dans un même plan horizontal. Les poutres étant nettement moins hautes que la paroi postérieure supérieure, la paroi postérieure peut pivoter autour d'un axe horizontal. La paroi postérieure supérieure peut s'incliner vers l'avant ou vers l'arrière grâce à cette construction. Cela améliore davantage l'adaptation morphologique au niveau du front. Ainsi, les froncements du front n'ont pas de répercussions sur les autres appuis du masque sur le visage.

Dans notre exemple, la quatrième paroi postérieure inférieure 2221d recouvrant le dessus du nez est reliée de manière relativement rigide à la partie antérieure. Cette quatrième paroi postérieure est donc peu mobile.

Selon un mode de réalisation, une des surfaces postérieures distinctes recouvre, ou est en appui sur, uniquement une partie du front, légèrement au-dessus des sourcils. La longueur L2221 de cette surface postérieure est au moins égale à la moitié de la distance entre les bords latéraux du châssis. Alternativement, outre une partie du front, cette surface recouvre, ou est en appui sur, une partie des pommettes. Cependant, cette surface postérieure distincte ne recouvre pas une partie du nez.

Dans cet exemple, le châssis n'est pas un élément de confort, il assure alors un maintien des éléments du masque. A ce titre, le châssis présente des propriétés mécaniques permettant d'assurer cette tenue. Le châssis est unitaire et composé d'un matériau dont la dureté est supérieure à 70 Shore A (mesurée selon ISO 868 et 7619, ASTM D 2240 et DIN 53505). Selon un mode de réalisation, le châssis est en PolyUréthane (PU) ce qui permet d'obtenir les caractéristiques précédentes recherchées. Dans ce cas, le châssis n'est pas constitué d'une mousse.

L'invention n'est pas limitée aux modes de réalisation précédemment décrits mais s'étend à tous les modes de réalisation couverts par les revendications annexées.

### Nomenclature

1- Masque
2- Châssis
   e2 Epaisseur châssis
   21- Attache latérale
   22- Corps
      221- Partie antérieure
      222- Partie postérieure
         2221 a, b, c, d- Paroi postérieure
         e2221 Epaisseur paroi postérieure
         h2221 Hauteur paroi postérieure
         L2221 Longueur paroi postérieure
         2222 a, b, c, d- Surface postérieure
         2223 Intervalle
         e2223 Profondeur intervalle
      223- Elément de liaison
3- Ecran
4- Sangle
5- Elément de confort

## Revendications

1. Masque de protection oculaire (1) pour la pratique de sports en plein air comprenant :
- un écran (3),
- un châssis (2) comprenant une partie antérieure (221) reliée à l'écran et une partie postérieure (222),
**caractérisé en ce que**
la partie postérieure du châssis comprend au moins deux surfaces postérieures (2222a, 2222b, 2222c) distinctes, chaque surface postérieure formant la face arrière d'une paroi postérieure (2221a, 2221b, 2222c) sensiblement verticale, les parois postérieures étant agencées de sorte que les surfaces postérieures associées forment une boucle discontinue entourant les deux yeux en recouvrant une partie du front, une partie des tempes et des pommettes et le dessus du nez, les parois postérieures étant espacées entre elles par un intervalle (2223) s'étendant, en direction de la partie antérieure du châssis, d'une profondeur (e2223) au moins égale à l'épaisseur (e2221) des parois postérieures délimitant l'intervalle.

2. Masque de protection oculaire (1) selon la revendication 1, **caractérisé en ce que** la partie postérieure (222) supporte au moins un élément de confort (5).

3. Masque de protection oculaire (1) selon la revendication 2, **caractérisé en ce que** chaque surface postérieure supporte un élément de confort distinct.

4. Masque de protection oculaire (1) selon la revendication 2, **caractérisé en ce qu'**un même élément de confort recouvre plusieurs surfaces postérieures.

5. Masque de protection oculaire (1) selon l'une des revendications précédentes, **caractérisé en ce qu'**une surface postérieure (2222a) forme uniquement une partie postérieure supérieure du châssis dont la largeur est au moins égale à la moitié de la largeur du châssis, ladite surface postérieure couvrant uniquement une partie du front.

6. Masque de protection oculaire (1) selon l'une des revendications précédentes, **caractérisé en ce que** la partie postérieure comprend au moins trois surfaces postérieures (2222a, 2222b, 2222c) distinctes agencées de la manière suivante :
- une surface supérieure (2222a) couvrant une partie du front,
- une surface latérale gauche (2222b) et une surface latérale droite (2222c) couvrant une partie des pommettes.

7. Masque de protection oculaire (1) selon l'une des revendications précédentes, **caractérisé en ce que** le châssis est unitaire et composé d'un matériau dont la dureté est supérieure à 70 Shore A.

8. Masque de protection oculaire (1) selon l'une des revendications précédentes, **caractérisé en ce que** le châssis est réalisé en PolyUréthane.

9. Masque de protection oculaire (1) selon l'une des revendications précédentes, **caractérisé en ce qu'**au moins un intervalle séparant deux parois postérieures s'étend d'une profondeur (e2223) au moins supérieure au quart de l'épaisseur (e2) du châssis, au niveau de cet intervalle.

10. Masque de protection oculaire (1) selon l'une des revendications précédentes, **caractérisé en ce que** la partie antérieure (221) et la partie postérieure (222) du châssis sont reliées par des éléments de liaison (223) dimensionnés de sorte à permettre le déplacement ou la déformation d'au moins une surface postérieure indépendamment des autres surfaces postérieures.

11. Masque de protection oculaire (1) selon la revendication précédente, **caractérisé en ce que** les moyens de liaison sont dimensionnés de sorte que l'amplitude du déplacement ou de la déformation d'au moins une surface postérieure est différente de celle d'une autre surface postérieure.

12. Masque de protection oculaire (1) selon l'une des deux revendications précédentes, **caractérisé en ce que** les éléments de liaison se présentent sous la forme d'un ensemble de poutres, les poutres étant inclinées par rapport à une direction linéaire normale à une surface postérieure et/ou formant une courbe ou un arc de cercle.

13. Masque de protection oculaire (1) selon l'une des trois revendications précédentes, **caractérisé en ce que** les éléments de liaison sont agencés de sorte à permettre un pivotement de la paroi postérieure sensiblement autour de son axe longitudinal.

14. Masque de protection oculaire (1) selon l'une des revendications précédentes, **caractérisé en ce que** chaque surface postérieure est allongée et sa longueur (L2221) est au moins quatre fois supérieure à sa hauteur (h2221).

15. Masque de protection oculaire (1) selon l'une des revendications précédentes, **caractérisé en ce que** l'épaisseur (e2221) de chaque paroi postérieure (2221) est au moins quatre fois inférieure à la hauteur (h2221) de cette paroi postérieure.

## Patentansprüche

1. Augenschutzbrille (1) zur Ausübung von Freiluftsport, die enthält:
- einen Schirm (3),
- ein Gestell (2), das einen mit dem Schirm verbundenen vorderen Teil (221) und einen hinteren Teil (222) enthält,
**dadurch gekennzeichnet, dass** der hintere Teil des Gestells mindestens zwei unterschiedliche hintere Flächen (2222a, 2222b, 2222c) enthält, wobei jede hintere Fläche die Rückseite einer im Wesentlichen senkrechten hinteren Wand (2221a, 2221b, 2221c) bildet, wobei die hinteren Wände so angeordnet sind, dass die verbundenen hinteren Flächen einen unterbrochenen Ring bilden, der die zwei Augen umgibt, indem er einen Teil der Stirn, einen Teil der Schläfen und Wangen und die Oberseite der Nase bedeckt, wobei die hinteren Wände um einen Zwischenraum (2223) zueinander beabstandet sind, der sich in Richtung des vorderen Teils des Gestells in einer Tiefe (e2223) mindestens gleich der Dicke (e2221) der den Zwischenraum begrenzenden hinteren Wände erstreckt.

2. Augenschutzbrille (1) nach Anspruch 1, **dadurch gekennzeichnet, dass** der hintere Teil (222) mindestens ein Komfortelement (5) trägt.

3. Augenschutzbrille (1) nach Anspruch 2, **dadurch gekennzeichnet, dass** jede hintere Fläche ein unterschiedliches Komfortelement trägt.

4. Augenschutzbrille (1) nach Anspruch 2, **dadurch gekennzeichnet, dass** das gleiche Komfortelement mehrere hintere Flächen bedeckt,

5. Augenschutzbrille (1) nach einem der vorhergehenden Ansprüche, **dadurch gekennzeichnet, dass** eine hintere Fläche (2222a) nur einen oberen hinteren Teil des Gestells bildet, dessen Breite mindestens gleich der Hälfte der Breite des Gestells ist, wobei die hintere Fläche nur einen Teil der Stirn bedeckt.

6. Augenschutzbrille (1) nach einem der vorhergehenden Ansprüche, **dadurch gekennzeichnet, dass** der hintere Teil mindestens drei unterschiedliche hintere Flächen (2222a, 2222b, 2222c) enthält, die folgendermaßen angeordnet sind:
- eine obere Fläche (2222a), die einen Teil der Stirn bedeckt,
- eine linke seitliche Fläche (2222b) und eine rechte seitliche Fläche (2222c), die einen Teil der Wangen bedecken.

7. Augenschutzbrille (1) nach einem der vorhergehenden Ansprüche, **dadurch gekennzeichnet, dass** das Gestell einstückig ist und aus einem Material besteht, dessen Härte größer als 70 Shore A ist.

8. Augenschutzbrille (1) nach einem der vorhergehenden Ansprüche, **dadurch gekennzeichnet, dass** das Gestell aus Polyurethan hergestellt wird.

9. Augenschutzbrille (1) nach einem der vorhergehenden Ansprüche, **dadurch gekennzeichnet, dass** mindestens ein zwei hintere Wände trennender Zwischenraum sich über eine Tiefe (e2223) mindestens größer als ein Viertel der Dicke (e2) des Gestells im Bereich dieses Zwischenraums erstreckt.

10. Augenschutzbrille (1) nach einem der vorhergehenden Ansprüche, **dadurch gekennzeichnet**, das der vordere Teil (221) und der hintere Teil (222) des Gestells durch Verbindungselemente (223) verbunden sind, die so bemessen sind, dass sie die Verschiebung oder die Verformung mindestens einer hinteren Fläche unabhängig von den anderen hinteren Flächen erlauben.

11. Augenschutzbrille (1) nach dem vorhergehenden Anspruch, **dadurch gekennzeichnet, dass** die Verbindungseinrichtungen so bemessen sind, dass die Amplitude der Verschiebung oder der Verformung mindestens einer hinteren Fläche sich von derjenigen einer anderen hinteren Fläche unterscheidet.

12. Augenschutzbrille (1) nach einem der zwei vorhergehenden Ansprüche, **dadurch gekennzeichnet, dass** die Verbindungselemente in Form einer Einheit von Trägern vorliegen, wobei die Träger bezüglich einer linearen Richtung normal zu einer hinteren Fläche geneigt sind und/oder eine Kurve oder einen Kreisbogen formen.

13. Augenschutzbrille (1) nach einem der drei vorhergehenden Ansprüche, **dadurch gekennzeichnet, dass** die Verbindungselemente so angeordnet sind, dass sie ein Schwenken der hinteren Wand im Wesentlichen um ihre Längsachse erlauben.

14. Augenschutzbrille (1) nach einem der vorhergehenden Ansprüche, **dadurch gekennzeichnet, dass** jede hintere Fläche länglich ist und ihre Länge (L2221) mindestens vier Mal größer ist als ihre Höhe (h2221).

15. Augenschutzbrille (1) nach einem der vorhergehenden Ansprüche, **dadurch gekennzeichnet, dass** die Dicke (e2221) jeder hinteren Wand (2221) mindestens vier Mal geringer als die Höhe (h2221) dieser hinteren Wand ist.

## Claims

1. Eye protection mask (1) for the practice of open-air sports, comprising:
- a screen (3),
- a chassis (2) comprising an anterior part (221) connected to the screen and a posterior part (222), **characterized in that**
the posterior part of the chassis comprises at least two separate posterior surfaces (2222a, 2222b, 2222c), each posterior surface forming the rear face of a substantially vertical posterior wall (2221a, 2221b, 2221c), the posterior walls being arranged such that the associated posterior surfaces form a discontinuous loop surrounding the two eyes while covering a part of the forehead, a part of the temples and the cheeks and the top of the nose, the posterior walls being spaced apart from one another by a gap (2223) extending, in the direction of the anterior part of the chassis, with a depth (e2223) at least equal to the thickness (e2221) of the posterior walls delimiting the gap.

2. Eye protection mask (1) according to Claim 1, **characterized in that** the posterior part (222) supports at least one comfort element (5).

3. Eye protection mask (1) according to Claim 2, **characterized in that** each posterior surface supports a separate comfort element.

4. Eye protection mask (1) according to Claim 2, **characterized in that** one and the same comfort element covers a plurality of posterior surfaces.

5. Eye protection mask (1) according to one of the preceding claims, **characterized in that** a posterior surface (2222a) forms only an upper posterior part of the chassis, the width of which is at least equal to half the width of the chassis, said posterior surface covering only a part of the forehead.

6. Eye protection mask (1) according to one of the preceding claims, **characterized in that** the posterior part comprises at least three separate posterior surfaces (2222a, 2222b, 2222c) arranged in the following manner:
- an upper surface (2222a) covering a part of the forehead,
- a left lateral surface (2222b) and a right lateral surface (2222c) covering a part of the cheeks.

7. Eye protection mask (1) according to one of the preceding claims, **characterized in that** the chassis is unitary and composed of a material whose hardness is greater than 70 Shore A.

8. Eye protection mask (1) according to one of the preceding claims, **characterized in that** the chassis is made of polyurethane.

9. Eye protection mask (1) according to one of the preceding claims, **characterized in that** at least one gap separating two posterior walls extends with a depth (e2223) at least greater than a quarter of the thickness (e2) of the chassis at this gap.

10. Eye protection mask (1) according to one of the preceding claims, **characterized in that** the anterior part (221) and the posterior part (222) of the chassis are connected by connection elements (223) dimensioned so as to allow the movement or the deformation of at least one posterior surface independently of the other posterior surfaces.

11. Eye protection mask (1) according to the preceding claim, **characterized in that** the connection means are dimensioned such that the amplitude of the movement or of the deformation of at least one posterior surface is different from that of another posterior surface.

12. Eye protection mask (1) according to one of the two preceding claims, **characterized in that** the connection elements take the form of a set of bars, the bars being inclined with respect to a linear direction normal to a posterior surface and/or forming a curve or an arc of a circle.

13. Eye protection mask (1) according to one of the three preceding claims, **characterized in that** the connection elements are arranged so as to allow a pivoting of the posterior wall substantially about its longitudinal axis .

14. Eye protection mask (1) according to one of the preceding claims, **characterized in that** each posterior surface is elongated and its length (L2221) is at least four times greater than its height (h2221).

15. Eye protection mask (1) according to one of the preceding claims, **characterized in that** the thickness (e2221) of each posterior wall (2221) is at least four times less than the height (h2221) of this posterior wall.
